# EUROPEAN PATENT SPECIFICATION

(11) **EP 4 469 995 B1**
(45) Date of publication and mention of the grant of the patent: **15.04.2026**
(21) Application number: 23716366.2
(22) Date of filing: 23.03.2023
(51) Int. Cl.: G09B 23/34, G09B 23/28

(54) **INTRAOPERATIVE ANATOMICAL LANDMARK IN THYROID SURGERY - RECURRENT LARYNGEAL NERVE INTERSTITIAL FLUID SHEATH**
INTRAOPERATIVE ANATOMISCHE LANDMARKEN IN DER SCHILDDRÜSENCHIRURGIE - REKURRENTE INTERSTITIELLE LARYNXNERVENHÜLLE
REPÈRE ANATOMIQUE PEROPÉRATOIRE DANS LA GAINE DE FLUIDE INTERSTITIEL DU NERF LARYNGÉ RÉCURRENT LORS DE LA CHIRURGIE THYROÏDIENNE

(30) Priority: 27.03.2022 EP 22398006
(43) Date of publication of application: 04.12.2024
(73) Proprietor: Revez Ferreira, Miguel Allen, 1750-441 LISBOA (PT)
(72) Inventor: Revez Ferreira, Miguel Allen, 1750-441 LISBOA (PT)
(74) Representative: Pereira da Cruz, Joao
(86) International application number: PCT/IB2023/052855
(87) International publication number: WO 2023/187558

(56) References cited:
- CN-A- 112 991 893
- RU-C1- 2 545 441
- US-A1- 2010 249 639
- US-A1- 2014 154 656
- US-A1- 2015 310 768
- GREGORY W. RANDOLPH ET AL: "Electrophysiologic recurrent laryngeal nerve monitoring during thyroid and parathyroid surgery: International standards guideline statement", THE LARYNGOSCOPE, vol. 121, no. S1, 22 December 2010 (2010-12-22), United States, pages S1 - S16, XP055487192, ISSN: 0023-852X, DOI: 10.1002/lary.21119

## Description

### FIELD OF THE INVENTION

The present disclosure relates generally to the field of surgical training. More specifically, the disclosure relates to an artificial 3D scaffold of thyroid and method of using the same for identification of the recurrent laryngeal nerve.

### BACKGROUND OF THE INVENTION

The prevalence of nodular thyroid pathology has increased over the last few years, mainly due to the higher number of diagnoses made by ultrasound. Population studies have shown an increase in the number of incidentally diagnosed thyroid nodules, with a subsequent increase in the number of aspiration cytology performed, resulting in part in increased diagnosis of thyroid cancer or suspected malignancy and, consequently, a greater number of thyroid surgeries performed.

Since the beginning of thyroid surgery, between 1873 and 1910, with Billroth, Kocher, Halsted and Quervain, the surgical technique of thyroidectomy has as main concerns: the hemóstase thorough, conservation of parathyroid ideas and avoid the injury of the recurrent laryngeal nerve (RLN). One of the main surgical risks is, therefore, the possible lesion of the recurrent laryngeal nerve, with the resulting transient or definitive dysphonia. Most patients with thyroid pathology are women of active age, with RLN injury having an important impact on quality of life. This will be most impactful the greater the need will be for a clear voice for the performance of their duties, particularly professionals.

Over the years, multiple studies and publications have been conducted with identification of several anatomical milestones that assist the surgeon in the identification of the RLN in his cervical path, namely using demonstrative photographs of embryological and anatomical aspects, emphasizing its clinical and surgical importance.

Even in the case of surgeons with a high volume of surgeries, whose experience is inversely proportional to the number of complications, the percentage of lesions of the RLN is not uncomplicated, recognized in the *most recent guidelines of* the American Thyroid Association (ATA).

Patent US20150310768A1 discloses a device for simulating at least one thyroid surgical procedure, the device comprising: a removable neck model configured to be accepted into a body model, the removable neck model comprising: a thyroid model comprising a first lobe and a second lobe connected by an isthmus; at least two first parathyroid gland models arranged posterior to the first lobe; at least two second parathyroid gland models arranged posterior to the second lobe; and a nerve model comprising a first superior laryngeal nerve model arranged medial and posterior to the first lobe, a second superior laryngeal nerve model arranged medial and posterior to the second lobe, a first recurrent laryngeal nerve model arranged medial and posterior to the first lobe, and a second recurrent laryngeal nerve model arranged medial and posterior to the second lobe.

Patent TWI711019B discloses a thyroid surgery training device, which uses a head simulation device to simulate the human head. The head simulation device includes a metal sensor on the inner side of one of the lower jaw members to simulate the mandibular nerve. The lower jaw part covers the lower lip part to simulate the skin of the lower lip. A neck simulation device is used to simulate the human neck, which is arranged under one of the lower jaw parts. The neck simulation device includes a neck body, a spine, a thyroid, a plurality of metal wires, and a plurality of blood vessels simulate the spine, thyroid, nerves and blood vessels in the human neck, and an electronic device is used to electrically connect the metal sensor and the metals. If the user gets too close or touches the metal wire, an alarm will be generated.

Patent WO2018088828A2 discloses a thyroid surgery region that may include a larynx model part disposed to extend in the forward and backward directions; a larynx region muscle model part surrounding at least a portion of the area around the larynx model part; a thyroid model part, at least a portion of which is interposed between the larynx model part and the larynx region muscle model part; and a larynx region-accommodating housing having a recessed part which is open on the top side and on the front and back, wherein the larynx model part, the larynx region muscle model part, and the thyroid model part are disposed in the recessed part.

As such, considering the foregoing, it may be appreciated that there is a need for novel methods for identification and visualization of RLN for its preservation during thyroidectomy.

### SUMMARY OF THE INVENTION

In one aspect of the disclosure, there is provided an artificial 3D scaffold of thyroid comprising artificial laryngeal and tracheal framework, wherein the scaffold comprises an artificial interstitial fluid sheath preferably with a viscosity from 2 cP to 8 cP.

In some embodiments, the artificial 3D scaffold comprises an artificial recurrent laryngeal nerve, the artificial recurrent laryngeal nerve preferably comprising a conducting wire, wherein the artificial interstitial fluid sheath encapsulates at least a portion of the artificial recurrent laryngeal nerve.

In some embodiments, the artificial 3D scaffold comprises an artificial vagus nerve, artificial vocal cords, wherein the artificial recurrent laryngeal nerve comprises a conducting wire, the conducting wire being in communication with the artificial vagus nerve and the artificial vocal cords.

In some embodiments, the artificial interstitial fluid sheath comprises a liquid encapsulating at least a portion of the artificial recurrent laryngeal nerve, and at least one outer layer, preferably at least two outer layers, encapsulating at least a portion of the liquid, wherein the layers are cuttable with a cutting device.

In some embodiments, the artificial 3D scaffold comprises artificial thyroid gland, artificial parathyroid, artificial trachea, artificial strap muscles, artificial internal jugular veins, artificial carotid arteries, artificial skin, artificial thyroid cartilages, artificial cricoid cartilages, artificial false capsule, artificial inferior thyroid artery, artificial superior pole vessels, artificial median thyroid vein, or any combination thereof.

In some embodiments, the artificial 3D scaffold is adjustable to an image of a lesion site.

In some embodiments, the artificial 3D scaffold is 3D printable.

In another aspect of the disclosure, there is provided an artificial 3D scaffold, for use in identification of the recurrent laryngeal nerve.

In another aspect of the disclosure, there is provided an artificial 3D scaffold, for use in surgery simulation and training.

In another aspect of the disclosure, there is provided a method for identification of the recurrent laryngeal nerve in a subject, comprising: a) providing a 3D model generated according to an image of a thyroid lesion site of a subject; b) fabricating an artificial 3D scaffold based on the 3D model; c) identifying an artificial interstitial fluid sheath in the artificial 3D scaffold; and d) opening the artificial interstitial fluid sheath, thereby visualizing and identifying the laryngeal nerve.

In some embodiments, the artificial 3D scaffold is the artificial 3D scaffold according to the present disclosure.

In some embodiments, identifying the artificial interstitial fluid sheath comprises cutting at least two outer layers, preferably at least three outer layers, wherein the layers encapsulate at least a portion of a liquid.

In some embodiments, the image of a lesion site is obtained from a camera, a magnetic resonance imaging (MRI), an X-ray image, a computed tomography (CT) scan, a 4D-CT scan, an image and report of thyroid and parathyroid ultrasounds, or any combination thereof.

In some embodiments, the step a) comprises creating a three-dimensional virtual reality (VR) computer model of a 3D scaffold, based on the image.

In some embodiments, the b) comprises printing the 3D scaffold using at least one three-dimensional printing device, or virtually simulate the 3D scaffold.

Unless otherwise defined, all technical and/or scientific terms used herein have the same meaning as commonly understood by one of ordinary skill in the art to which the disclosure pertains. Although methods and materials similar or equivalent to those described herein can be used in the practice or testing of embodiments of the disclosure, exemplary methods and/or materials are described below. In case of conflict, the patent specification, including definitions, will control. In addition, the materials, methods, and examples are illustrative only and are not intended to be necessarily limiting.

Further embodiments and the full scope of applicability of the present disclosure will become apparent from the detailed description given hereinafter.

### BRIEF DESCRIPTION OF THE DRAWINGS

Some embodiments of the disclosure are herein described, by way of example only, with reference to the accompanying drawings. With specific reference now to the drawings in detail, it is stressed that the particulars shown are by way of example and for purposes of illustrative discussion of embodiments of the disclosure. In this regard, the description taken with the drawings makes apparent to those skilled in the art how embodiments of the disclosure may be practiced.
Figure 1 is an anatomical illustration of an anteroposterior view of artificial 3D scaffold comprising artificial recurrent laryngeal nerve (13), preferably comprising a conducting wire, in communication with the artificial vagus nerve (10) and artificial vocal cords (16);
Figure 2 is an anatomical illustration of a lateral view of an artificial 3D scaffold according to the present disclosure, showing the location of the interstitial fluid sheath (30) within which, an artificial recurrent laryngeal nerve (13) comprising a conducting wire conductor passes, and its connection to an artificial vagus nerve (10) and artificial vocal cords (16);
Figure 3 is an anatomical illustration of a lateral view an artificial 3D scaffold according to the present disclosure, and corresponding cross sections showing the location of an artificial interstitial fluid sheath (30) surrounding the artificial RLN (13);
Figure 4 is an anatomical illustration of a detail cut of an artificial 3D scaffold according to the present disclosure, representing outer layers (15), encapsulating an artificial interstitial fluid sheath (30); and
Figure 5 is an anatomical illustration of some of the components of an artificial 3D scaffold according to the present disclosure, including the location of the artificial interstitial fluid sheath (30) that encapsulates the artificial recurrent laryngeal nerve (13).

### DETAILED DESCRIPTION OF THE INVENTION

According to some embodiments, the present disclosure provides an artificial 3D scaffold of thyroid comprising artificial laryngeal and tracheal framework. According to the present disclosure, an artificial 3D scaffold as described herein, is used as a 3D simulator of thyroid and parathyroid surgery.

According to the present disclosure, an artificial 3D scaffold as described herein, comprises an artificial interstitial fluid sheath. According to the present disclosure, an artificial 3D scaffold as described herein, comprises an artificial recurrent laryngeal nerve (RLN).

An artificial 3D scaffold as described herein, is a highly reliable simulator for training surgeons in thyroid and parathyroid surgery in order to reduce the learning curve. The artificial 3D scaffold can be used as a training device for surgeons in thyroid surgery, improving surgeons' thyroid surgery proficiency, and reducing recurrent laryngeal nerve damage.

The present disclosure is based, in part, on the fact that an artificial 3D scaffold as described herein, is able to simulate the consistency of different organs or tissues, vessels and nerves. This is possible by choosing and/or tuning the materials used in the scaffold and in the different components. Non-limiting examples of such materials include polymeric materials, silicone with different densities and rigidity, polyethylene, polyurethane foam, resins, rigid polyurethane materials, elastic soft resin, Nylon powder, collagen, cellulose, silk, keratin, fibrous materials and nonwoven.

The present disclosure is based, in part, on the fact that an artificial 3D scaffold as described herein, comprising an artificial interstitial fluid sheath, is able to mimic the interstitial fluid sheath present in the human body, that encapsulates the RLN. This is achieved by choosing a material with an appropriate viscosity and/or composition. In some embodiments, an artificial interstitial fluid sheath as described herein comprises a viscosity from 2 centipoise (cP) to 8 cP, preferably from 2 cP to 4 cP. The technical effect obtained by an artificial interstitial fluid sheath that is able to mimic the interstitial fluid sheath present in the human body, is that the mimicking allows for a realistic training of surgeons using the 3D scaffold as described herein.

In some embodiments, an artificial interstitial fluid sheath as described herein comprises a pH from 6.5 to 7.5.

In some embodiments, the artificial interstitial fluid sheath comprises an aqueous solution.

In some embodiments, the artificial interstitial fluid sheath comprises NaCl, preferably at a concentration from 100 mM to 200 mM; KCl, preferably at a concentration from1 mM to 8 mM; MgCl₂ preferably at a concentration from 0.2 mM to 5 mM; MgSO₄, preferably at a concentration from 0.2 mM to 5 mM; NaHCO₃, preferably at a concentration from 1 mM to 100 mM; CaCl₂, preferably at a concentration from 2 mM to 20 mM; KH₂PO₄, preferably at a concentration from 0.5 mM to 10 mM, or any combination thereof.

In some embodiments, the artificial interstitial fluid sheath comprises a buffer, preferably at a concentration of about 20 mM. In some embodiments, the buffer is selected from the group consisting of DPBS, Tris, Tris-HCI, Tris buffered saline, or PBS.

In some embodiments, the artificial interstitial fluid sheath comprises a simulated body fluid. In some embodiments, simulated body fluid refers to simulated interstitial fluid (SIF).

As used herein, the terms "simulated body fluid" (SBF) and "simulated interstitial fluid" (SIF) are used interchangeably and refer to a solution with an ion concentration close to that of human blood plasma, kept under mild conditions of pH and identical physiological temperature. SBF are usually defined as acellular, protein-free, supersaturated calcium-phosphate solutions with ionic compositions almost equal to the composition of human blood plasma generally buffered at physiological conditions (pH = 7.4 and 36.5 °C).

In some embodiments, the artificial 3D scaffold comprises at least one material selected from a polymeric material, silicone with different densities and rigidity, polyethylene, polyurethane foam, resins, rigid polyurethane materials, elastic soft resin, Nylon powder, collagen, cellulose, silk, keratin, fibrous materials, nonwoven, or any combination thereof.

In some embodiments, the artificial 3D scaffold comprises live-wire material. According to the present disclosure, the presence of live-wire material (such as conductive wires) allows for simulating the identification of the artificial recurrent laryngeal nerve, with neuromonitoring equipment.

According to some embodiments, the present disclosure provides an artificial 3D scaffold of thyroid comprising artificial laryngeal and tracheal framework, wherein the scaffold comprises an artificial interstitial fluid sheath.

As used herein, the term "interstitial fluid sheath" refers to a fluid present between the true and false capsule of the thyroid, that surrounds the recurrent laryngeal nerve in its cervical course, posterior to the thyroid lobe. During the medial mobilization of the thyroid lobe, keeping a sharp dissection between the true and false capsule of the thyroid, especially when using loop magnification and avoiding suction and sponges to dry the surgical field, a sheath encasing interstitial fluid is possible to identify.

As used herein, the term "laryngeal and tracheal framework" refers to the anatomic structures of larynx, and trachea, and their corresponding anatomic position. Non-limiting example of anatomic structures of the framework according to the present disclosure include membranes, nerves, cartilages, muscles, vocal cords, ligaments, bones, vertebrae, and any combination thereof.

In some embodiments, the artificial 3D scaffold comprises an artificial recurrent laryngeal nerve (RLN).

According to the present disclosure, an interstitial fluid sheath involves (encapsulates) the RLN (Interstitial RLN Fluid Sheath), as can be demonstrated in thyroid dissection. The identification of an Interstitial Fluid Sheath serves as an anatomical landmark for early identification of the RLN. During the medial mobilization of the thyroid lobe, keeping the sharp dissection between the true and false capsule of the thyroid, a sheath encasing interstitial fluid is possible to identify. The opening of this sheath immediately reveals the presence of the artificial recurrent laryngeal nerve, with its extra-laryngeal branching.

The present disclosure is based, in part, on the fact that an artificial 3D scaffold as described herein comprising an artificial interstitial fluid sheath and an artificial recurrent laryngeal nerve (RLN), allows for training surgeons in identification and visualization of RLN allowing its preservation and reducing the risk of damage to the RLN, during thyroidectomy.

In some embodiments, the artificial recurrent laryngeal nerve comprises a conducting wire. In some embodiments, the artificial 3D scaffold comprises an artificial vagus nerve, artificial vocal cords, or both. In some embodiments, the artificial 3D scaffold comprises an artificial vagus nerve, artificial vocal cords, and an artificial recurrent laryngeal nerve comprising a conducting wire, wherein the conducting wire is in communication with the artificial vagus nerve and the artificial vocal cords.

The present disclosure is based, in part, on the finding that an artificial 3D scaffold comprising a conducting wire is able to simulate the visualization, identification and/or contact with the recurrent laryngeal nerve. The conducting wire is connected to the vagus nerve, and the vocal cords, allowing an electromyography identification with a system of intraoperative neuromonitoring.

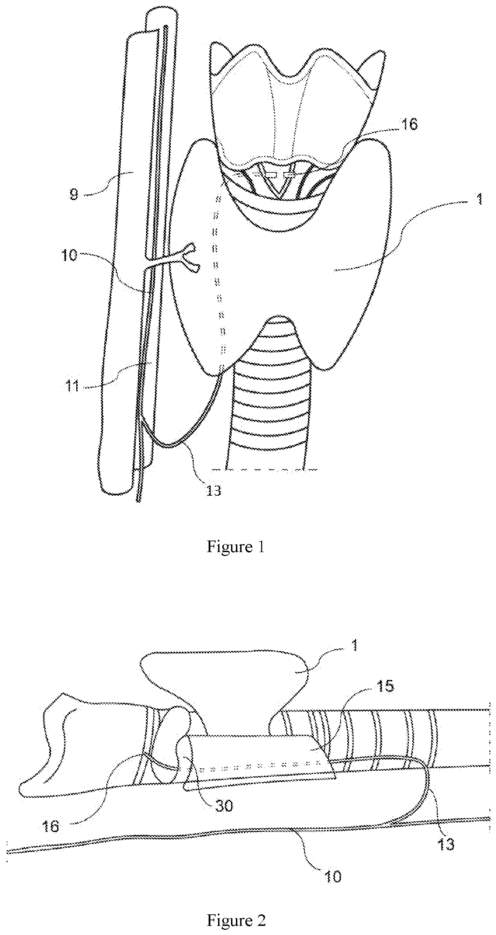

Reference is made to Figure 1 and Figure 2 that represent anatomical illustration of an anteroposterior view (Figure 1) and lateral view (Figure 2) of an artificial 3D scaffold according to the present disclosure, showing the location of the interstitial fluid sheath (30) within which, an artificial recurrent laryngeal nerve (13), preferably comprising a conducting wire is positioned, and its connection to an artificial vagus nerve (10) and artificial vocal cords (16).

According to Figure 1, an artificial 3D scaffold comprises an artificial thyroid gland (1) and an artificial recurrent laryngeal nerve (13), preferably comprising a conducting wire, in communication with an artificial vagus nerve (10), and artificial vocal cords (16). The artificial vagus nerve (10) is positioned between an artificial jugular vein (9) and an artificial carotid artery (11).

Reference is made to Figure 2, which is a lateral view of an artificial 3D scaffold according to the present disclosure. In some embodiments, an artificial 3D scaffold comprises an artificial thyroid gland (1) and an artificial recurrent laryngeal nerve (13), preferably comprising a conducting wire, in communication with an artificial vagus nerve (10), and artificial vocal cords (16). The artificial 3D scaffold comprises an artificial interstitial fluid sheath (30) encapsulating at least a portion of the artificial recurrent laryngeal nerve (13), and at least two outer layers (15), preferably at least three outer layers (15) encapsulating the artificial interstitial fluid sheath (30) and the artificial recurrent laryngeal nerve (13).

In some embodiments, an artificial 3D scaffold comprises an artificial laryngeal and tracheal framework, wherein the scaffold comprises an artificial interstitial fluid sheath (30) preferably with a viscosity from 2 cP to 8 cP.

In some embodiments, an artificial 3D scaffold comprises an artificial recurrent laryngeal nerve (13), preferably comprising a conducting wire, wherein the artificial interstitial fluid sheath (30) encapsulates at least a portion of the artificial recurrent laryngeal nerve (13), as described in Figure 2.

In some embodiments, an artificial 3D scaffold comprises at least two outer layers (15), preferably at least three outer layers (15) encapsulating the artificial interstitial fluid sheath and the artificial recurrent laryngeal nerve (13).

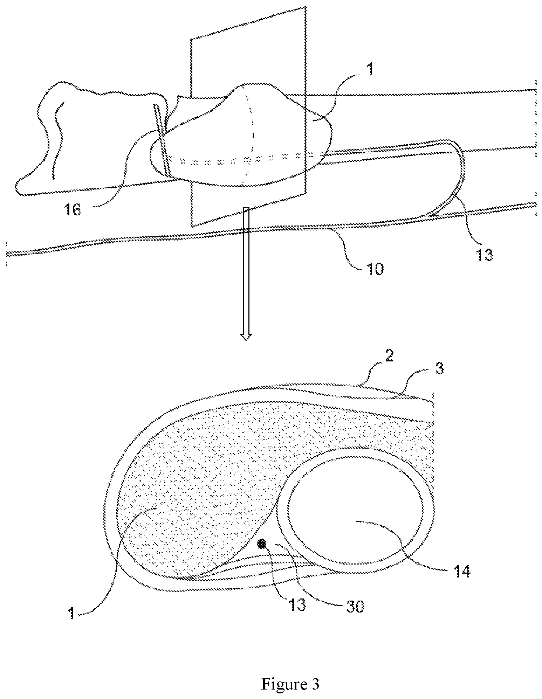

Reference is made to Figure 3 which presents an anatomical illustration of a lateral view of an artificial 3D scaffold according to the present disclosure, and corresponding cross sections showing the location of an artificial interstitial fluid sheath (30) surrounding the artificial RLN (13). In some embodiments, an artificial interstitial fluid sheath (30) is located between an artificial thyroid gland (1) and an artificial trachea (14). In some embodiments, an artificial 3D scaffold according to the present disclosure, comprises a superficial aspect of an anterior layer of Pretracheal fascia (2) and deep aspect of an anterior layer of Pretracheal fascia (3).

In some embodiments, the artificial interstitial fluid sheath (30) comprises a liquid encapsulating at least a portion of the artificial recurrent laryngeal nerve (13), and at least two outer layers (15), preferably at least three outer layers (15), encapsulating at least a portion of the liquid, wherein the layers are cuttable with a cutting device. Suitable cutting devices are well known in the art and will become apparent to a skilled person in the art. Non-limiting examples of cutting devices according to the present disclosure include scalpels, scissors, drills, burs, saws, and rongeurs.

In some embodiments, the layers comprise an easily cuttable material. As used herein, an easily cuttable material, refers to a material that can be easily cut with a cutting device as described hereinabove. In some embodiments, the layers comprise an elastomer. Elastomer are loosely cross-linked polymers and are well known in the art. Non-limiting examples of elastomers include natural rubbers, styrene-butadiene block copolymers, polyisoprene, polybutadiene, ethylene propylene rubber, ethylene propylene diene rubber, silicone elastomers, fluoroelastomers, polyurethane elastomers, and nitrile rubbers. In some embodiments, the layers comprise silicon, preferably translucid silicone.

In some embodiments, the layers comprise imprinted drawings of microvessels. According to the present disclosure these imprinted drawing of microvessels represent microvessels that have to be coagulated in real-life surgery to maintain a blood-free surgical field.

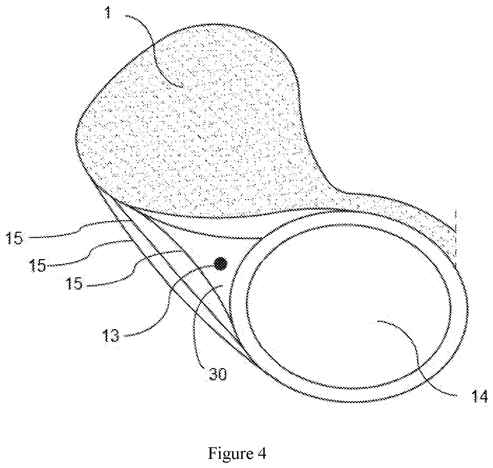

Reference is made to Figure 4 which is an anatomical illustration of a detail cut of an artificial 3D scaffold according to the present disclosure, representing outer layers (15), encapsulating an artificial interstitial fluid sheath (30).

In some embodiments, the artificial 3D scaffold according the present disclosure, comprises artificial thyroid gland, artificial parathyroid, artificial trachea, artificial strap muscles, artificial internal jugular veins, artificial carotid arteries, artificial skin, artificial thyroid cartilages, artificial cricoid cartilages, artificial false capsule, artificial inferior thyroid artery, artificial superior pole vessels, artificial median thyroid vein, or any combination thereof. The anatomy and location of the components described hereinabove as part of the present model are well known in the art and their location relative to each other and in the artificial 3D scaffold would become apparent to a skilled person in the art.

In some embodiments, an artificial 3D scaffold comprises and artificial thyroid gland, an anterior layer of Pretracheal fascia, a deep aspect of an anterior layer of Pretracheal fascia, an artificial true thyroid capsule, an artificial sternohyoid muscle, artificial sternocleidomastoid muscle, artificial skin flap, artificial jugular vein, artificial vagus nerve, artificial carotid artery, artificial parathyroid gland, an artificial interstitial fluid sheath, an artificial trachea, or any combination thereof.

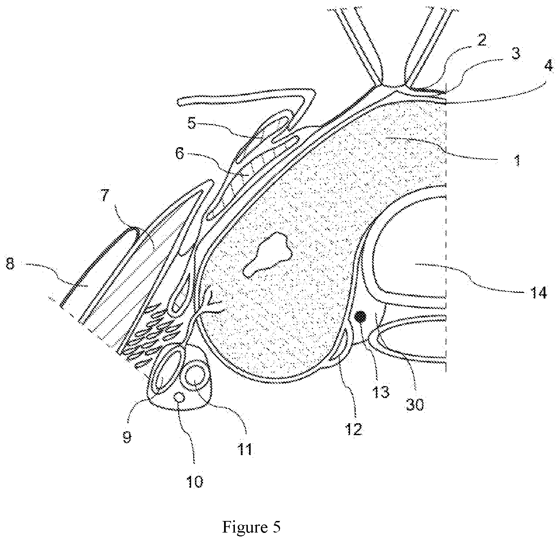

Reference is made to Figure 5 which is an anatomical illustration of some of the components of and artificial 3D scaffold according to the present disclosure.

In some embodiments, an artificial 3D scaffold comprises and artificial thyroid gland (1), an artificial anterior layer of Pretracheal fascia (2), an artificial deep aspect of an anterior layer of Pretracheal fascia (3), an artificial true thyroid capsule (4), an artificial sternohyoid muscle (5, 6), artificial sternocleidomastoid muscle (7), artificial skin flap (8), artificial jugular vein (9), artificial vagus nerve (10), artificial carotid artery (11), artificial parathyroid gland (12), artificial recurrent laryngeal nerve (13), an artificial interstitial fluid sheath (30), and an artificial trachea (14).

In some embodiments, the artificial 3D scaffold is adjustable to an image of a lesion site. In some embodiments, the image of a lesion site is obtained from a camera, a magnetic resonance imaging (MRI), an X-ray image, a computed tomography (CT) scan, a 4D-CT scan, an image and report of thyroid and parathyroid ultrasounds, or any combination thereof.

In some embodiments, the artificial 3D scaffold is 3D printable.

In some embodiments, the artificial 3D scaffold is personalized to a subject. According to some embodiments of the present disclosure, the artificial 3D scaffold is personalized to a subject, by using e.g., results of CT exams of the subject, performed in the pre-operatory, to fabricate the artificial 3D scaffold.

In some embodiments, the artificial 3D scaffold as described herein, is for use in the identification of the recurrent laryngeal nerve. In some embodiments, there is disclosed a use of the artificial 3D scaffold as described herein for use in the identification of the recurrent laryngeal nerve.

In some embodiments, the artificial 3D scaffold as described herein, is for use in surgery simulation and training. In some embodiments, there is disclosed a use of the artificial 3D scaffold as described herein for surgery simulation and training. According to the present disclosure, surgery simulation and training comprises simulation of thyroid surgery, training in thyroid surgery, simulation with intraoperative neuromonitoring equipment, training with intraoperative neuromonitoring equipment, and simulation prior to a given thyroid or parathyroid surgery on a subject, based on their preoperative exams.

According to the present disclosure, there is provided a method for identification of the recurrent laryngeal nerve in a subject. In some embodiments, the method comprises providing an artificial 3D scaffold as described herein; visualizing the artificial interstitial fluid sheath; and opening the artificial interstitial fluid sheath, thereby visualizing and identifying the simulated laryngeal nerve.

In some embodiments, opening the artificial interstitial fluid sheath comprises cutting the outer layers.

According to the present disclosure, there is provided a method for identification of the recurrent laryngeal nerve in a subject, comprising: a) providing a 3D model generated according to an image of a thyroid lesion site of a subject; b) fabricating an artificial 3D scaffold based on the 3D model; c) identifying the artificial interstitial fluid sheath in the artificial 3D scaffold; and d) opening the artificial interstitial fluid sheath, thereby visualizing and identifying the simulated laryngeal nerve.

In some embodiments, the artificial 3D scaffold is the artificial 3D scaffold as described hereinabove.

In some embodiments, identifying the artificial interstitial fluid sheath comprises cutting at least one outer layer, preferably at least two outer layers, wherein the layers encapsulate at least a portion of a liquid.

In some embodiments, the image of a lesion site is obtained from a camera, a magnetic resonance imaging (MRI), an X-ray image, a computed tomography (CT) scan, a 4D-CT scan, an image and report of thyroid and parathyroid ultrasounds, or any combination thereof.

In some embodiments, step a) comprises creating a three-dimensional virtual reality (VR) computer model of a 3D scaffold, based on the image.

In some embodiments, step b) comprises printing the 3D scaffold using at least one three-dimensional printing device, or virtually simulate the 3D scaffold.

In some embodiments, preceding the step of visualizing the artificial interstitial fluid sheath, the method comprises the following steps: 1) artificial skin incision, preferably 2 cm above a manubrium notch; 2) opening of a surgical field by separation of strap muscles in a midline; 3) medial mobilization of a thyroid lobe, by means of sharp dissection between a true and false capsule of the thyroid (2 layers with interstitial space in between); 4) identification and ligation of a median thyroid vein; 5) if a system of intraoperative neuromonitoring is available, monitor a vagus nerve, between a jugular vein and a carotid artery; 6) a sharp dissection, in a number of at least 1, at least 2 of preferably at least 3 similar sheaths (or layers) with imprinted drawings of microvessels, that are identifiable before the interstitial fluid sheath. The layers are to be dissected individually, simulating the real-life surgery to maintain a blood-free surgical field. The above-mentioned layers are placed in a matter that permits individual dissection/ opening, before reaching the interstitial fluid sheath that encases a fluid/ gel and contains the artificial laryngeal nerve.

In some embodiments, the method comprises the following steps: 7) an inferior thyroid artery is identifiable, and ligated.

In some embodiments, the interstitial fluid sheath, comprising a translucid liquid or gel, that involves a conducting wire simulating the recurrent laryngeal nerve, is opened, resulting in spillage of the fluid and leading to visualization of the laryngeal nerve.

In some embodiments, if a system of intraoperative neuromonitoring is available, it is used to monitor the recurrent laryngeal nerve. The conducting wire (simulating the recurrent laryngeal nerve) connected to the vagus nerve and to the vocal folds, allows to perform the electromyographic identification.

According to the present disclosure, there is provided a method for obtaining a 3D scaffold as disclosed herein. In some embodiments, the method comprises providing a 3D model generated according to an image of a thyroid lesion site; and fabricating a 3D scaffold based on the 3D model.

In some embodiments, the image of a lesion site is obtained from a camera, a magnetic resonance imaging (MRI), an X-ray image, a computed tomography (CT) scan, a 4D-CT scan, an image and report of thyroid and parathyroid ultrasounds, or any combination thereof.

In some embodiments, fabricating comprises printing the 3D scaffold using at least one three-dimensional printing device, or virtually simulate the 3D scaffold.

In some embodiments, fabricating comprises using at least one polymeric material.

As used herein the term "about" refers to ± 10 %.

The terms "comprises", "comprising", "includes", "including", "having" and their conjugates mean "including but not limited to".

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the disclosure may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable subcombination or as suitable in any other described embodiment of the disclosure. Certain features described in the context of various embodiments are not to be considered essential features of those embodiments, unless the embodiment is inoperative without those elements.

### EXAMPLES

Reference is now made to the following examples, which together with the above descriptions illustrate some embodiments of the disclosure in a non-limiting fashion.

### Materials and methods

### Observational Prospective Longitudinal Study

Patients diagnosed with thyroid pathology with surgical indication, identified in a surgical consultation and confirmed in a multidisciplinary consultation (malignant and suspected malignancy), who meet the inclusion and exclusion criteria, will be recruited for the study.

After explaining the concept and objectives of the study to patients and after confirmation that all their doubts have been clarified, an informed consent is signed. All individuals will be observed in a preoperative evaluation consultation, with videolaryngoscopy in a defined protocol.

Each of the 150 to 200 consecutive dissections of RLN at risk will be recorded on ultra-HD video, demonstrating the attempt at systematic identification in each case of the presence of the Interstitial Fluid Sheath involving the RLN.

The surgeon is always the same (the investigator), the two helpers are always the same (co-investigators) and the classification as to the ease of identification of the Interstitial Fluid Sheath that involves the RLN will be performed by the surgeon in the course of each surgery, by consensus with the surgical team, to assess the sensitivities.

It will be rated 0, Unidentified; 1, Slightly present; 2, Easily identified; 3, Exuberant.

Being an anatomical visual landmark, its demonstration must be performed using video imaging, and the eventual subjectivity of identification is evaluated by the agreement/disagreement of the classification of several observers.

To evaluate the degree of intra- and interobserver agreement, identification and classification, all videos will be observed one month later, with concealment, without knowledge of the case to which they belong and how it was classified intraoperatively. This second evaluation will be made individually by the surgeon and by each of the assistants, and the three answers are only known when evaluating the results.

In a second phase, three surgeons will be invited, with high experience in thyroid surgery, but with no experience of identifying this structure, to evaluate the videos of all cases and classify according to the four categories identified (videos of representative samples of each category will be previously provided).

Although it is not the gold standard (there is no gold standard beyond the visual identification of the RLN) in thyroid surgery, in all cases intraoperative neuromonitoring will be performed, according to the International Intraoperative Monitoring Study Group, as it is the only way to demonstrate, with formal registration [latency (time - 3.96 ms (±0.69) and wave amplitude - 891 µV(±731)]) the immediate identification of the driving corresponding to the RLN - R1.

All patients will also be evaluated, as is regular clinical practice, by otorhinolaryngologist in voice consultation, in the back and one month after surgery, with videolaryngoscopy, for direct evaluation of vocal cord mobility, in order to be able to demonstrate possible organic, neurological or traumatic lesions.

All patients will be evaluated for their hydration degree, using Bioelectric Impedance Vector Analysis (BIVA), in order to try to demonstrate a possible association between the degree of hydration and the ease of identification of the Interstitial Fluid Sheath that surrounds the RLN.

The value will be made preoperatively, in the seven days preceding the surgery, without any intervention, serving only, in this phase, to try to correlate the degree of hydration with greater or lesser ease of identification of the BLI.

Although there is preoperative intravenous hydration, the fluids to be administered depends on the needs of each case and cannot be controlled. However, and in the case of elective surgical, from anesthetic induction to recurrent laryngeal nerve, only about 20 minutes take place, so it is not considered to significantly alter the volume of the 3rd^{a} space.

Preoperative Bioimpedance may, demonstrating its correlation, suggest an indication for future change in the preoperative preparation of patients undergoing thyroid surgery, which will in itself be a new study with intervention.

Similarly, the chronic medicines that patients are taking, including diuretics, may be a relevant data of the assessment of the degree of hydration because they reduce it, and will be a variable to be assessed.

### EXAMPLE 1

The present research led to the identification of a new anatomical landmark, not previously described, in thyroid surgery- the interstitial fluid sheath of the recurrent laryngeal nerve (RLN).

Being able to demonstrate its existence and usefulness as an anatomical landmark, it will have as consequence the easier identification of the RLN and for this reason, combining with all the remaining surgical technique and other anatomical landmarks described, can help in the reduction of the iatrogenic lesions of the nerve.

### Potential scientific and societal impact

Alteration of the surgical technique in order to facilitate the identification of the RLN by the prior identification of the Interstitial Fluid Sheath of the RLN (with dissection using avascular embryonic planes, with magnifying loupes and sealing and cutting equipment).

Change of preoperative preparation with hydration of patients in order to facilitate the identification of the RLN interstitial fluid sheath.

It reduces the morbidity of thyroid surgery related to RLN injuries with consequent reduction of health expenses and medical leaves (social security), reduction of legal disputes.

Creation of dedicated courses of cervical endocrine surgery, oriented to expert and non-expert surgeons, to teach and disseminate the possibility of using this anatomical landmark to minimize potential iatrogenic lesions of the recurrent laryngeal nerve.

### EXAMPLE 2

Surgery execution requires training, repetition and volume to gain competence.

The creation of an artificial 3D anatomic model of thyroid comprising artificial laryngeal and tracheal framework, and comprising the anatomical landmark, the interstitial fluid sheath of the recurrent laryngeal nerve (RLN), allows for the training of surgeons in the identification of this landmark thereby reducing possible injuries in the RLN.

Although the disclosure has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. Artificial 3D scaffold of thyroid comprising artificial laryngeal and tracheal framework, **characterized in that** said scaffold comprises an artificial interstitial fluid sheath (30) and an artificial recurrent laryngeal nerve (13), wherein said artificial interstitial fluid sheath (30) is surrounding said artificial recurrent laryngeal nerve (13) and preferably has a viscosity from 2 cP to 8 cP.

2. Artificial 3D scaffold according to claim 1, wherein said artificial recurrent laryngeal nerve (13) preferably comprises a conducting wire, and said artificial interstitial fluid sheath (30) encapsulates at least a portion of said artificial recurrent laryngeal nerve (13).

3. Artificial 3D scaffold according to claim 2, comprising an artificial vagus nerve (10), artificial vocal cords (16), wherein said artificial recurrent laryngeal nerve (13) comprises a conducting wire, said conducting wire being in communication with said artificial vagus nerve (10) and said artificial vocal cords (16).

4. Artificial 3D scaffold according to any one of claims 1 to 3, wherein said artificial interstitial fluid sheath (30) comprises a liquid encapsulating at least a portion of said artificial recurrent laryngeal nerve (13), and at least one outer layer (15), preferably at least two outer layers (15), encapsulating at least a portion of said liquid, wherein said layers are cuttable with a cutting device.

5. Artificial 3D scaffold according to any one of claims 1 to 4, comprising artificial thyroid gland (1), artificial parathyroid, artificial trachea (14), artificial strap muscles, artificial internal jugular veins, artificial carotid arteries, artificial skin, artificial thyroid cartilages, artificial cricoid cartilages, artificial false capsule, artificial inferior thyroid artery, artificial superior pole vessels, artificial median thyroid vein, or any combination thereof.

6. Artificial 3D scaffold according to any one of claims 1 to 5, wherein said artificial 3D scaffold is adjustable to an image of a lesion site.

7. Artificial 3D scaffold according to any one of claims 1 to 6, wherein said 3D scaffold is 3D printable.

8. Artificial 3D scaffold according to any one of claims 1 to 7, for use in identification of the recurrent laryngeal nerve.

9. Artificial 3D scaffold according to any one of claims 1 to 8, for use in surgery simulation and training.

10. Method for identification of the recurrent laryngeal nerve in a subject, **characterized in** by comprising the following steps:
a) providing a 3D model generated according to an image of a thyroid lesion site of a subject;
b) fabricating an artificial 3D scaffold based on said 3D model;
c) identifying an artificial interstitial fluid sheath (30) surrounding an artificial recurrent laryngeal nerve (13) in said artificial 3D scaffold; and
d) opening said artificial interstitial fluid sheath (30), thereby visualizing and identifying the artificial recurrent laryngeal nerve (13).

11. Method according to claim 10, wherein said artificial 3D scaffold is the artificial 3D scaffold according to any one of claims 1 to 9.

12. Method according to claim 10 to 11, wherein identifying the artificial interstitial fluid sheath (30) comprises cutting at least two outer layers (15), preferably at least three outer layers (15), wherein said layers encapsulate at least a portion of a liquid.

13. Method according to any one of claims 10 to 12, wherein said image of a lesion site is obtained from a camera, a magnetic resonance imaging (MRI), an X-ray image, a computed tomography (CT) scan, a 4D-CT scan, an image and report of thyroid and parathyroid ultrasounds, or any combination thereof.

14. Method according to any one of claims 10 to 13, wherein step a) comprises creating a three-dimensional virtual reality (VR) computer model of a 3D scaffold, based on said image.

15. Method according to any one of claims 10 to 14, wherein step b) comprises printing said 3D scaffold using at least one three-dimensional printing device, or virtually simulate said 3D scaffold.

## Patentansprüche

1. Künstliches 3D-Schilddrüsengerüst, das ein künstliches Kehlkopf- und Luftröhrengerüst umfasst, **dadurch gekennzeichnet, dass** das Gerüst eine künstliche interstitielle Flüssigkeitshülle (30) und einen künstlichen rekurrenten Kehlkopfnerv (13) umfasst, wobei die künstliche interstitielle Flüssigkeitshülle (30) den künstlichen rekurrenten Kehlkopfnerv (13) umgibt und vorzugsweise eine Viskosität von 2 cP bis 8 cP aufweist.

2. Künstliches 3D-Gerüst nach Anspruch 1, wobei der künstliche rekurrente Kehlkopfnerv (13) vorzugsweise einen leitenden Draht umfasst und die künstliche interstitielle Flüssigkeitshülle (30) mindestens einen Teil des künstlichen rekurrenten Kehlkopfnervs (13) einkapselt.

3. Künstliches 3D-Gerüst nach Anspruch 2, umfassend einen künstlichen Vagusnerv (10), künstliche Stimmbänder (16), wobei der künstliche rekurrente Kehlkopfnerv (13) einen leitenden Draht umfasst, wobei der leitende Draht in Verbindung mit dem künstlichen Vagusnerv (10) und den künstlichen Stimmbändern (16) steht.

4. Künstliches 3D-Gerüst nach einem der Ansprüche 1 bis 3, wobei die künstliche interstitielle Flüssigkeitsumhülle (30) eine Flüssigkeit, die mindestens einen Teil des künstlichen rekurrenten Kehlkopfnervs (13) einkapselt, und mindestens eine äußere Schicht (15), vorzugsweise mindestens zwei äußere Schichten (15), die mindestens einen Teil der Flüssigkeit einkapseln, umfasst, wobei die Schichten mit einer Schneidvorrichtung schneidbar sind.

5. Künstliches 3D-Gerüst nach einem der Ansprüche 1 bis 4, umfassend eine künstliche Schilddrüse (1), eine künstliche Nebenschilddrüse, eine künstliche Luftröhre (14), künstliche Gurtmuskeln, künstliche innere Jugularvenen, künstliche Halsschlagadern, künstliche Haut, künstliche Schilddrüsenknorpel, künstliche Krikoidknorpel, eine künstliche falsche Kapsel, eine künstliche untere Schilddrüsenarterie, künstliche obere Polgefäße, eine künstliche mittlere Schilddrüsenvene oder eine beliebige Kombination davon.

6. Künstliches 3D-Gerüst nach einem der Ansprüche 1 bis 5, wobei das künstliche 3D-Gerüst auf ein Bild einer Läsionsstelle einstellbar ist.

7. Künstliches 3D-Gerüst nach einem der Ansprüche 1 bis 6, wobei das 3D-Gerüst 3D-druckbar ist.

8. Künstliches 3D-Gerüst nach einem der Ansprüche 1 bis 7 zur Verwendung bei der Identifizierung des rekurrenten Kehlkopfnervs.

9. Künstliches 3D-Gerüst nach einem der Ansprüche 1 bis 8, zur Verwendung in der chirurgischen Simulation und Ausbildung.

10. Verfahren zur Identifizierung des rekurrenten Kehlkopfnervs in einer Person, **dadurch gekennzeichnet, dass** es die folgenden Schritte umfasst:
a) Bereitstellen eines 3D-Modells, das anhand eines Bildes einer Schilddrüsenläsionsstelle eines Patienten erzeugt wird;
b) Herstellen eines künstlichen 3D-Gerüsts auf der Grundlage des 3D-Modells;
c) Identifizieren einer künstlichen interstitiellen Flüssigkeitsumhülle (30), die einen künstlichen rekurrenten Kehlkopfnerv (13) in dem künstlichen 3D-Gerüst umgibt;
und
d) Öffnen der künstlichen interstitiellen Flüssigkeitshülle (30), wodurch der künstliche rekurrente Kehlkopfnerv (13) sichtbar gemacht und identifiziert wird.

11. Verfahren nach Anspruch 10, wobei das künstliche 3D-Gerüst das künstliche 3D-Gerüst nach einem der Ansprüche 1 bis 9 ist.

12. Verfahren nach einem der Ansprüche 10 bis 11, wobei das Identifizieren der künstlichen interstitiellen Flüssigkeitshülle (30) das Schneiden von mindestens zwei äußeren Schichten (15), vorzugsweise mindestens drei äußeren Schichten (15), umfasst, wobei die Schichten mindestens einen Teil einer Flüssigkeit einkapseln.

13. Verfahren nach einem der Ansprüche 10 bis 12, wobei das Bild einer Läsionsstelle von einer Kamera, einem Magnetresonanztomographen (MRI), einem Röntgenbild, einem Computertomographen (CT), einem 4D-CT-Scan, einem Bild und Bericht von Schilddrüsen- und Nebenschilddrüsen-Ultraschall oder einer beliebigen Kombination davon erhalten wird.

14. Verfahren nach einem der Ansprüche 10 bis 13, wobei Schritt a) das Erstellen eines dreidimensionalen Virtual-Reality (VR)-Computermodells eines 3D-Gerüsts auf der Grundlage des Bildes umfasst.

15. Verfahren nach einem der Ansprüche 10 bis 14, wobei Schritt b) das Drucken des 3D-Gerüstes unter Verwendung mindestens einer dreidimensionalen Druckvorrichtung oder die virtuelle Simulation des 3D-Gerüstes umfasst.

## Revendications

1. Matrice 3D artificielle de la thyroïde comprenant une structure laryngée et trachéale artificielle, **caractérisée en ce que** ladite matrice comprend une gaine artificielle de fluide interstitiel (30) et un nerf laryngé récurrent artificiel (13), dans laquelle ladite gaine artificielle de fluide interstitiel (30) entoure ledit nerf laryngé récurrent artificiel (13) et a de préférence une viscosité comprise entre 2 cP et 8 cP.

2. Matrice 3D artificielle selon la revendication 1, dans laquelle ledit nerf laryngé récurrent artificiel (13) comprend de préférence un fil conducteur, et ladite gaine artificielle de fluide interstitiel (30) encapsule au moins une partie dudit nerf laryngé récurrent artificiel (13).

3. Matrice 3D artificielle selon la revendication 2, comprenant un nerf vague artificiel (10) et des cordes vocales artificielles (16), dans laquelle ledit nerf laryngé récurrent artificiel (13) comprend un fil conducteur, ledit fil conducteur étant en communication avec ledit nerf vague artificiel (10) et lesdites cordes vocales artificielles (16).

4. Matrice 3D artificielle selon l'une quelconque des revendications 1 à 3, dans laquelle ladite gaine artificielle de fluide interstitiel (30) comprend un liquide encapsulant au moins une partie dudit nerf laryngé récurrent artificiel (13), et au moins une couche externe (15), de préférence au moins deux couches externes (15), encapsulant au moins une partie dudit liquide, lesdites couches étant sectionnables à l'aide d'un dispositif de coupe.

5. Matrice 3D artificielle selon l'une quelconque des revendications 1 à 4, comprenant une glande thyroïde artificielle (1), une parathyroïde artificielle, une trachée artificielle (14), des muscles sous-hyoïdiens artificiels, des veines jugulaires internes artificielles, des artères carotides artificielles, une peau artificielle, des cartilages thyroïdiens artificiels, des cartilages cricoïdes artificiels, une fausse capsule artificielle, une artère thyroïdienne inférieure artificielle, des vaisseaux du pôle supérieur artificiels, une veine thyroïdienne médiane artificielle, ou toute combinaison de ceux-ci.

6. Matrice 3D artificielle selon l'une quelconque des revendications 1 à 5, ladite matrice 3D artificielle étant ajustable à une image d'un site lésionnel.

7. Matrice 3D artificielle selon l'une quelconque des revendications 1 à 6, ladite matrice 3D étant imprimable en 3D.

8. Matrice 3D artificielle selon l'une quelconque des revendications 1 à 7, destinée à l'identification du nerf laryngé récurrent.

9. Matrice 3D artificielle selon l'une quelconque des revendications 1 à 8, destinée à la simulation et à l'entraînement chirurgicaux.

10. Procédé d'identification du nerf laryngé récurrent chez un sujet, **caractérisé en ce qu'**il comprend les étapes suivantes :
a) fournir un modèle 3D généré à partir d'une image d'un site lésionnel thyroïdien dudit sujet ;
b) fabriquer une matrice 3D artificielle sur la base dudit modèle 3D ;
c) identifier une gaine artificielle de fluide interstitiel (30) entourant un nerf laryngé récurrent artificiel (13) dans ladite matrice 3D artificielle ; et
d) ouvrir ladite gaine artificielle de fluide interstitiel (30), permettant ainsi de visualiser et d'identifier le nerf laryngé récurrent artificiel (13).

11. Procédé selon la revendication 10, dans lequel ladite matrice 3D artificielle est la matrice 3D artificielle selon l'une quelconque des revendications 1 à 9.

12. Procédé selon l'une quelconque des revendications 10 à 11, dans lequel l'identification de la gaine artificielle de fluide interstitiel (30) comprend couper au moins deux couches externes (15), de préférence au moins trois couches externes (15), lesdites couches encapsulant au moins une partie d'un liquide.

13. Procédé selon l'une quelconque des revendications 10 à 12, dans lequel ladite image d'un site lésionnel est obtenue à partir d'une caméra, d'une imagerie par résonance magnétique (IRM), d'une image radiographique, d'une tomodensitométrie (CT), d'une tomodensitométrie 4D, d'une image et d'un rapport d'échographie thyroïdienne et parathyroïdienne, ou de toute combinaison de ceux-ci.

14. Procédé selon l'une quelconque des revendications 10 à 13, dans lequel l'étape a) comprend la création d'un modèle informatique tridimensionnel en réalité virtuelle (VR) d'une matrice 3D, sur la base de ladite image.

15. Procédé selon l'une quelconque des revendications 10 à 14, dans lequel l'étape b) comprend l'impression de ladite matrice 3D au moyen d'au moins un dispositif d'impression tridimensionnelle, ou la simulation virtuelle de ladite matrice 3D.
